# EUROPEAN PATENT SPECIFICATION

(11) **EP 1 511 843 B1**
(45) Date of publication and mention of the grant of the patent: **29.03.2006**
(21) Application number: 03757145.2
(22) Date of filing: 06.06.2003
(51) Int. Cl.: C12N 15/10, C12Q 1/68, G01N 33/50, C12M 1/34

(54) **SCREENING METHODS**
SCREENING-VERFAHREN
PROCEDES DE CRIBLAGE

(30) Priority: 07.06.2002 US 386391 P; 07.06.2002 DK 200200869
(43) Date of publication of application: 09.03.2005
(73) Proprietor: Sophion Bioscience A/S, 2750 Ballerup (DK)
(72) Inventor: JESPERSEN, Thomas, Kobenhavns Universitet, DK-2200 Kobenhavn N (DK); BECH, Morten, Sophion Bioscience A/S, DK-2750 Ballerup (DK); KUTCHINSKY, Jonatan, Sophion Bioscience A/S, DK-2750 Ballerup (DK); OLESEN, Soren, Peter, DK-2930 Klampemborg (DK); TABORYSKI, Rafael, Sophion Bioscience A/S, DK-2750 Ballerup (DK)
(74) Representative: Johnstone, Helen Margaret
(86) International application number: PCT/GB2003/002459
(87) International publication number: WO 2003/104455

(56) References cited:
- EP-A- 1 143 013
- WO-A-01/25769
- WO-A-01/59447
- WO-A-01/73000
- WO-A-02/04943
- WO-A-02/24862
- WO-A-02/29402
- WO-A-97/27212
- WO-A-99/64582
- DE-A- 19 950 385

## Description

The present invention relates to screening methods and, in particular, to methods of screening DNA libraries to identify DNA molecules which, when expressed in a host cell, give rise to at least one change in the phenotype of the host cell.

DNA libraries enable the convenient storage and analysis of polynucleotides, and their transcribed (mRNA) and expressed products (peptides, polypeptides or proteins). The term "DNA library" used herein is intended to embrace a collection of DNA molecules, wherein the individual members of the library encode a different single gene and/or fragment and/or variant thereof. Alternatively, the individual members of the library encode different genes and/or fragments and/or variants thereof.

Screening of DNA libraries conventionally directly investigates the heterologous DNA sequence and/or its expression product with little or no consideration of its effect on cells. Additionally most known methods of DNA library screening are not rapid, high throughput, easy to use techniques.

WO 02/24862 discloses systems for positioning and/or analyzing samples such as cells, vesicles, cellular organelles, and fragments, derivatives, and mixtures thereof, for electrical and/or optical analysis, especially relating to the presence and/or activity of ion channels.

WO 02/04943 discloses apparatuses for measuring cell membrane resistance, conductance, potential, and capacitance, which can be used to determine cellular electrical activity. In particular, automated apparatuses are designed for high-throughput analysis of various compounds, ligands, and cell processes that modulate cellular electrical properties (e.g., cell membrane conditions). The automated apparatuses are used for the determination of ion channel activity, and for the rapid identification of compounds, ligands, or processes that alter this activity. WO'943 also discloses methods for measuring cellular electrical properties utilizing the apparatuses provided therein.

WO 99/64582 discloses high-throughput screening means and methods to determine the function of the product(s) of one or more sample nucleic acids. The sample nucleic acids were synthetic oligonucleotides, DNA, or cDNA and encode polypeptides, antisense nucleic acids or GSEs. The sample nucleic acids were expressed in a host by a vehicle to alter at least one phenotype of the host. The altered phenotype(s) was identified as a means to assign a biological function to the product(s) encoded by the sample nucleic acid(s).

WO 01/73000 discloses methods for identifying and controlling the genetic and metabolic pathways underlying complex phenotypes. Conjoint polynucleotide segments that contributed to or disrupted elements of a multigenic phenotype were produced and expressed in cells of interest. Conjoint polynucleotide segments were recombined and/or mutated to give rise to libraries of recombinant concatamers which were expressed in cells of interest. Libraries of conjoint polynucleotide segments and recombinant concatamers were expressed episomally or integrated into the DNA of organelles or chromosomes. Cells were screened or selected to identify members of the population of cells exhibiting a desired phenotype. Libraries and vectors comprising conjoint polynucleotide segments and recombinant concatamers, as well as cells expressing such libraries and vectors or their components were provided. Kits containing conjoint polynucleotide segments, recombinant concatamers, vectors including such polynucleotides, and cells including such polynucleotides and vectors were provided.

EP 1 143 013 discloses compositions and methods directed at screening or characterizing compounds that modulate the activity of calcium channels in cells preferably, calcium-release activated channels in cells. The compositions and methods are used to produce inhibitors or activators of the channel, which represent leads or candidate therapeutic drugs for treating various pathological conditions. The method comprises the steps of: (a) contacting a test compound and a calcium channel activator, preferably an Icrac activator with a population of calcium channel expressing cells, preferably Icrac expressing cells containing a reporter construct comprising a reporter gene under the control of a NFAT-inducible promoter, and (b) determining the activity of the test compound on the calcium release-activated channel by assessing the reporter gene expression in the cells.

R. Gehwolf *et al.* in their paper entitled 'First Patch, then catch: measuring the activity and the mRNA transcripts of a proton pump in individual Lilium pollen protoplasts' (FEBS Letters, Vol. 512, pages 152-156, 13 February 2003), disclose combining the patch-clamp method with single-cell reverse transcription polymerase reaction (scRT-PCR). A fusicoccin-induced current reflecting the activity of the plasma membrane H⁺ ATPase of lily pollen protoplasts was measured and subsequently, the ATPase-encoding mRNAs were collected and amplified. Southern bolt signals were observed in all 'patch-catch' experiments and could be detected even in 2560-fold dilutions of the pollen contents. H⁺ ATPase mRNAs were detectable only in vegetative but not in generative cell of pollen as confirmed by immunolocalisation.

According to the invention there is provided a method for screening a DNA library comprising;
(i) providing a substrate for making electrophysiological measurements upon which at least one cell can be arranged;
(ii) providing a plurality of cells, each cell comprising a different heterologous DNA sequence derived from a DNA library, wherein each cell expresses the heterologous DNA sequence it comprises;
(iii) arranging the plurality of cells provided in step (ii) on the substrate to permit detection and/or measurement of a change (in comparison to a control cell) in the electrophysiology of each cell, said change being a result of expression of the heterologous DNA sequence; and
(iv) identifying at least one cell of interest which shows at least one phenotypic change.

Screening DNA libraries in the method of this invention allows the close study of nucleotide sequences of known and unknown function, including genes and their expressed peptides, polypeptides and proteins in association with the cellular phenotype.

Preferably, the cell and/or genetic material of interest is isolated to enable further study of the heterologous DNA e.g. by sequencing.

Preferably, the results of further studies are maintained on an information carrier. Most preferably, the information carrier is one selected from one or more of the following: paper, email, computer disk and internet page.

Preferably, screening of a cell population expressing a given DNA library is performed by electrophysiological measurements and/or fluorescence microscopy and/or microscopic analyses of changes in cellular volume and/or shape.

The method of the present invention is used to perform electrophysiological measurements.

The principle of studying ion channels in a small membrane area by electrically insulating said patch of membrane under voltage-clamp conditions was outlined by Neher, Sakmann, and Steinback in "The Extracellular Patch Clamp, A Method For Resolving Currents Through Individual Open Channels In Biological Membranes", Pflueger Arch. 375; 219-278, (1978). They found that, by pressing a pipette containing acetylcholine (ACh) against the surface of a muscle cell membrane, they could see discrete jumps in electrical current that were attributable to the opening and closing of ACh-activated ion channels. Their work was limited by the fact that the resistance of the seal between the glass of the pipette and the membrane (10-50 MΩ) was very small relative to the resistance of the channel (10 GΩ). The electrical noise resulting from such a seal is large enough to obscure the currents flowing through ion channels.

It was then discovered that by fire polishing the glass pipettes and by applying suction to the interior of the pipette a seal of very high resistance (1-100 GΩ) could be obtained with the surface of the cell. This Giga-seal reduced the noise by an order of magnitude to levels at which most channels of biological interest can be studied and greatly extended the voltage range over which these studies could be made. This improved seal has been termed a "giga-seal", and the pipette has been termed a "patch pipette". A more detailed description of the giga-seal may be found in O.P. Hamill, A. Marty, E. Neher, B. Sakmann & F.J. Sigworth: Improved patch-clamp techniques for high resolution current recordings from cells and cell-free membrane patches. Pflugers Arch. 391, 85-100, (1981).

Ion channels are transmembrane proteins catalysing transport of inorganic ions across cell membranes. Ion channels are involved in many cellular processes including generating and timing action potentials, synaptic transmission, secretion of hormones, contraction of muscles. Many drugs exert their specific effects via modulation of ion channels. Examples include the antiepileptic compound phenytoin which blocks voltage-dependent Na⁺-channels in the brain and the antihypertensive drug nifedipine which blocks voltage dependent Ca²⁺ -channels in smooth muscle cells. In addition to chemically induced modulation of ion-channel activity, the patch clamp technique has enabled scientists to perform manipulations with voltage dependent channels. These techniques include adjusting the polarity of the electrode in the patch pipette and altering the saline composition to moderate the free ion levels in the bath solution.

Preferably, screening for phenotype changes can further be conducted by studying the effects of the heterologous DNA sequence in conjunction with an applied intra- and/or extra- cellular test agent.

Most preferably, the test agent is at least one selected from one or more of the following: small organic molecules (MW < 1000 g/mol), small peptides (less than 100 amino acids), neurotransmitters, hormones and cytokines.

Preferably, the cell containing the heterologous DNA sequence is an animal cell, most preferably selected from Human Embryonic Kidney 293 (HEK 293), Chinese Hamster Ovary (CHO), COS, MDCK, NG108, NIH3T3 or T84.

When an extracellular (e.g. ion concentration, temperature or ligand protein concentration) or intracellular (e.g. ion concentration, protein concentration or a heterologous protein) component is altered a change in the cellular conditions may occur. The presence of a recombinant DNA sequence can therefore influence cellular conditions and the measurable phenotype. Predominantly the influence is on the intracellular component wherein the recombinant DNA is expressed thus altering protein concentrations and via the function of some of these proteins, influencing other cellular processes. The influence of the expressed heterologous protein can also extend to effects on extracellular conditions.

An example of the influence of the expressed recombinant protein influencing cellular conditions occurs when the expressed recombinant protein is a cell membrane ion channel. Both the intracellular and extracellular conditions are affected. An ion channel allows and/or causes the movement of ions across cellular membranes. This movement is either extra- to intra-cellular or vice versa and therefore changes the ion concentration both intra- and extra-cellularly.

These changes in cellular conditions are phenotypic changes that can be measured and used to indicate the presence of a genotype of interest i.e. the recombinant DNA encodes a genotype causing a measurable change in the host cell phenotype. The phenotypic changes are measured in comparison to control cells derived from the same cell line as those host cells being transfected, differing in that the control cells are not transfected with a recombinant DNA sequence.

The change in host cell phenotype can be measured using the chip apparatus as described herein, whereby the host cell is subjected to phenotype testing whilst in contact with the substrate of the chip.

Construction of cellular expressed DNA libraries for use in the invention can be performed by standard PCR and cloning techniques in combination with retroviral vector technology. Each member of the DNA library is introduced into host cells such that each cell comprises only one recombinant DNA construct. The presence of two or more heterologous recombinant constructs weakens any potential observable phenotype signal and characterisation of the causal genotype for the observed phenotype is made more difficult. Stable integration of a single recombinant construct in each cell can be achieved by using a low multiplicity of infection (MOI) of the retroviral particles, ensuring a low level of infection of host cells and combined with selection for a resistance gene, contained within the retroviral vector. Preferably the resistance gene used for selection is one from: Neomycin, puromycin, blasticidin D or zeomycin.

Preferred examples of DNA libraries to be studied are cDNA, randomised, polymorphism and genomic libraries.

By "cDNA libraries" we mean DNA libraries obtained by reverse transcription of cellular mRNA.

Advantageously, normalised cDNA libraries are used in order to eliminate a bias from highly expressed genes, and by normalised cDNA libraries we mean cDNA libraries wherein high frequency cDNAs are reduced in frequency producing an approximately equal representation of cDNAs.

By "randomised libraries" we mean DNA libraries of a single polynucleotide wherein each member of the library differs from each other member by one or more nucleotides and said variation has been artificially introduced.

By "polymorphism libraries" we mean DNA libraries of a single polynucleotide wherein each member of the library differs from each other member by one or more nucleotides, wherein said variation is a natural and inherent feature of that polynucleotide.

By "genomic libraries" we mean DNA libraries obtained by the fractionation of whole cellular genomic DNA.

DNA libraries can be constructed by several different molecular technologies. Complementary DNA (cDNA) libraries, the most commonly used DNA libraries, are constructed from mRNA isolated from eukaryotic cells. The structure of mRNA incorporates a 3' poly A tail added during post-transcriptional processing, enabling cDNA to be synthesised from the processed mRNA. Isolated mRNA is incubated with an excess of poly T oligonucleotides and reverse transcriptase enzymes, wherein the poly T oligonucleotide anneals to the complementary poly A tail. The reverse transcriptase enzyme is then able to use the poly T oligonucleotide as a primer from which to initiate DNA synthesis whereby the mRNA acts as the template to produce single-stranded DNA for subsequent use as the template for double-stranded cDNA synthesis. The cDNA can be utilised in standard PCR reactions or used for cloning into vectors to create a DNA library [F M Ausubel et al. "Current protocols in Molecular Biology - Volume One" John Wiley & sons, Inc. (1995)].

Having been synthesised from mRNA, cDNA differs from genomic DNA in that it does not contain regulatory sequences or introns. The cDNA produced represents the mRNA content of a cell at a particular time, and is unlikely to equally represent every gene from the genome of the parent cell.

Hence, cDNA libraries contain a biased representation of the cellular mRNA, predominantly consisting of housekeeping genes in multiple copies, and low expression genes present in few or no copies. Furthermore, there are unexpressed genes that will not be among the cDNAs produced.

It is possible, through a normalisation procedure to create cDNA libraries with a more equal representation of cellular mRNAs [M B *Soares et al.* "Construction and characterisation of a normalised cDNA library", PNAS 91 pp.9228-32 (1994)]. This procedure is performed by using the complementary single-stranded cDNA as an annealing partner and subsequent selection of free single-stranded cDNA. This methodology ensures that high frequency cDNAs are predominantly associated with the complementary strand and considerably reduced in frequency, while normally low frequency cDNAs are only moderately reduced. Selection in this manner can be repeated to produce an approximately equal representation of cDNAs.

A further DNA library, is a genomic DNA library constructed by the direct fractionisation of cellular genomic DNA and insertion of the resulting fragments into an appropriate vector. Such libraries contain DNA with noncoding regions intact. Typically, the fractionation of genomic DNA is achieved by mechanical or enzymatic means.

A further type of DNA library is a randomised library, wherein the library contains clones encoding a single gene, each clone containing a different modification of one or more nucleotides, typically but not exclusively corresponding to the same specific peptide domain of the protein under investigation. Such randomised libraries may be constructed using PCR technology [T Ohmichi *et al.* "Efficient bacterial transcription of DNA nanocircle vectors with optimised single-stranded promoters", PNAS 99 pp.54-59 (2002); T Jespersen *et al.* "Efficient non-PCR mediated overlap extension of PCR fragments by exonuclease "end-polishing", Biotechniques 23 pp.48-52 (1997)]. The randomised modification(s) are introduced into the target nucleotide via PCR primers incorporating the randomised nucleotide sequence(s), thus targeting the modification(s) to specific amino acid positions.

A yet further type of DNA library is a polymorphic library. Many genes within the population of a given organism are polymorphic, i.e. natural variants of the same gene encoding a particular protein. For example, Major Histocompatability Complex (MHC) proteins contain polymorphisms of amino acids within the peptide binding cleft, such that the binding cleft differs, thereby accounting for the variation between individuals in the ability of their MHC proteins to bind specific targets. [P J Bjorkman and P Parham "Structure, function and diversity of class I Major Histocompatability Complex molecules" Ann. Rev. Biochem. 59 pp. 253-288 (1990)].

A polymorphic library therefore comprises each member of the library encoding a different polymorphic copy of a single gene. Such libraries are particularly of benefit for drug screening to study differentiated response patterns and potential side effects associated with particular polymorphic forms of a gene.

Methods of introducing the DNA into cells are well known in the art [ Sambrook & Russell, "Molecular cloning; A laboratory manual" 3^{rd} edition, Cold Spring Harbor Laboratory Press (2001)] and include electroporation, liposome mediated transfer, calcium phosphate co-precipitation and microinjection.

Electroporation is achieved by the application of a brief electrical pulse to a solution containing DNA and the cells that are to be transfected [Review - K Shigekawa & W J Dower "Electroporation of eukaryotes and prokaryotes: a general approach to the introduction of macromolecules into cells", Biotechniques 6 pp. 742-751 (1988)]. The electrical pulse causes transient permeabilisation of the cell membrane during which DNA is able to enter the cell cytosol. A fraction of this DNA is subsequently found in the nucleus where transient expression of the introduced genes occurs, typically peaking between 24 and 72 hours after transfection.

Liposome mediated transfer (lipofection) utilises vesicles with artificial lipid membranes resembling natural lipid cell membranes (liposomes), to carry recombinant DNA. The liposome is able to fuse spontaneously to cell membranes, thereby releasing their contents into the cell cytoplasm. [Review - R J Mannino and S Gould-Fogerite, " Liposome mediated gene transfer", Biotechniques, 6 pp. 682-690 (1988)]

Calcium Phosphate co-precipitation was shown by F L Braham and A J van der Eb " A new technique for the assay of infectivity of human adenovirus 5 DNA" Virol., 52 pp. 456-467 (1973); to greatly increase gene transfer into host cells. DNA is mixed with a phosphate buffer solution, to which calcium chloride is added. This produces a fine precipitate of calcium phosphate and DNA that is applied to the host cells, and the cells take up the precipitated DNA into their cytosol over several hours.

Recombinant DNA is reliably introduced into host cells by the process of microinjection. This method requires a fine needle to penetrate the cell, and the DNA is injected directly into the cell cytosol [M Capecchi, " High efficiency transformation by direct microinjection into cultured mammalian cells" Cell, 22 pp. 479-488 (1980)]. Microinjection can be performed with computer controlled apparatus that increases the precision and speed of the technique.

The DNA sequence introduced to the target cell is heterologous DNA, by which we mean a DNA sequence that has been introduced to the cell over and above the cell's normal DNA contents, and wherein each heterologous DNA member of the DNA library differs from each other member by one or more nucleotides.

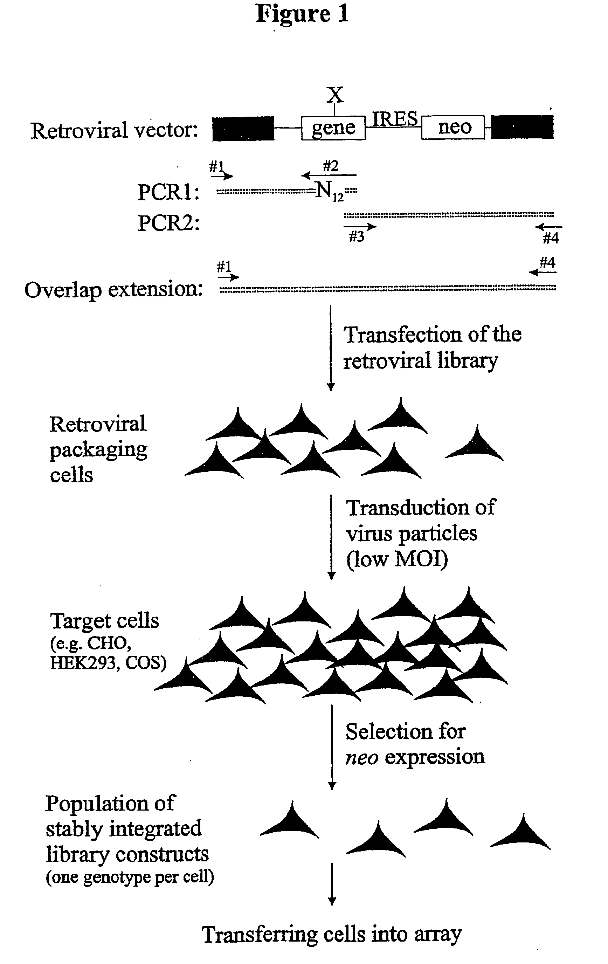

Reliable expression of the heterologous DNA sequence is achieved by stable integration of the cloned DNA into the host cell's DNA. This is reliably achieved, for example, using retroviral transduction systems based upon the natural ability of retroviruses to stably integrate their genome into the host cell's DNA [Mann et al. "Construction of a retrovirus packaging mutant and its use to produce helper-free defective retrovirus", Cell 33 pp.153-159 (1983)] {Figure 1}. In such a system the cloned DNA is introduced into the viral genome. Retroviral transduction systems conventionally contain two functional elements, cis-elements and trans-elements. The trans-elements, encode viral packaging proteins; and cis-elements encode other essential viral nucleotide sequences. Cloned DNA is introduced into the host genome such that it is surrounded by cis-elements as recombinant proviral DNA that is unable to produce its own packaging proteins, therefore the proviral DNA is transfected into a packaging cell.

Packaging cells contain the trans-element and produce the viral coats into which the cloned proviral DNA is packaged to produce infectious particles.

The infectious particles are released from the host cell and can be harvested for transfecting the target cell culture, in which it stably integrates the viral genome including the cloned DNA contained therein. The cloned DNA can therefore be expressed in the target cell, and the retrovirus is unable to further replicate as the recombinant viral genome lacks the necessary viral genes [Review - J M Coffin "Retroviridiae: The viruses and their replication, pp. 1767 - 1845" In : D M Knipe *et al.* (ed.) "Fields virology" Lippencott-Raven publishers (1996)].

### Description of the Drawings

Specific embodiments of the invention will now be described with reference to the figures described below:
Figure 1 *- Schematic diagram showing construction and cellular expression of a DNA library using retroviral vectors.* The vector contains the heterologous DNA of interest (gene), a resistance marker (neo - neomycin resistance) and an internal ribosome entry site (IRES). The heterologous DNA is flanked by cis- elements, which include the long terminal repeats (LTR). Transcription of this construct produces mRNA containing both the heterologous DNA and the resistance marker. Randomisation of a specific sequence is achieved by PCR followed by overlap extension. Primer #2 contains randomised nucleotides to introduce nucleotide changes at specific places. The fragments obtained from PCR are mixed in overlap extension to obtain a full-length vector for the retroviral vector transduction system. The vector constructs are transfected into packaging cells where transient expression produces supernatant that when extracted contains virus that can be applied to the target cells.

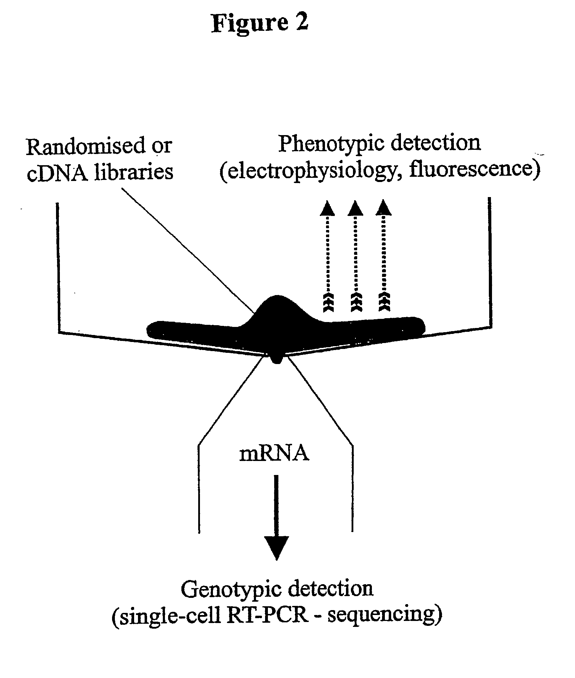

Figure 2 *- Schematic diagram showing a single target cell positioned in the test site on the chip.* The cell expresses a single genomic construct. Below the cell is a small opening between the test site and well, the cell membrane at this site is perforated or removed for electrophysiological analysis. Other phenotype analyses are shown (dotted lines). Genotype detection is also conducted by extraction of the mRNA via the opening over which the cell membrane is incomplete.

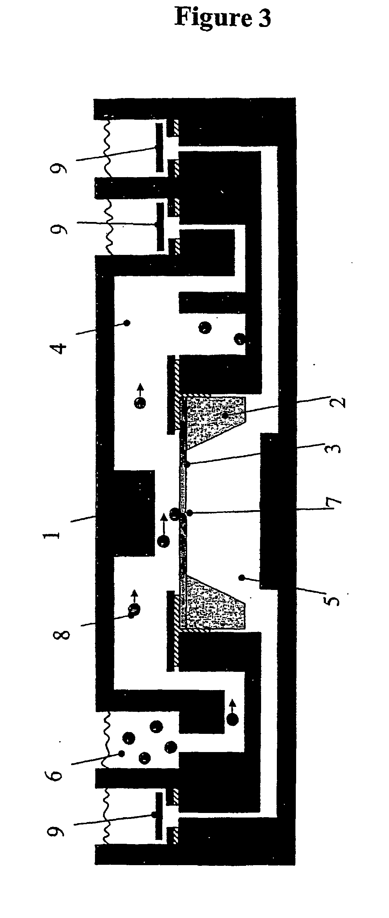

Figure 3 - Schematic diagram of the preferred chip construct. Constructed with:
   1. Chip housing.
   2. Micro structured unit.
   3. Glass/silica membrane on micro structured unit.
   4. Cell containing channel/compartment containing extracellular buffer solution and compound.
   5. Channel containing intracellular buffer solution and mRNA from cell in whole-cell configuration.
   6. Inlet port/pipetting well for introduction of cells and compound.
   7. Cell capture site consisting of an orifice in the membrane 3. The orifice is appropriately shaped to enable a gigaseal and whole-cell formation.
   8. Cell in suspension.
   9. Micropumps.

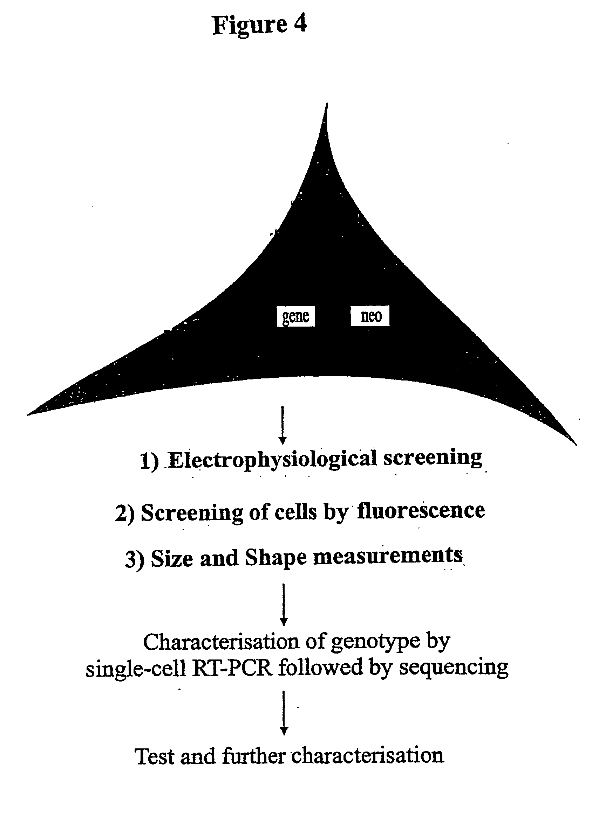

Figure 4 - *Schematic diagram showing methods of screening cell phenotype.* A cell with a stably integrated vector for expressing cDNA, from which a membrane bound protein is produced (e.g. ion channel).
Figure 5 - *Schematic diagram showing phenotype investigation of a peptide randomisation DNA library.* Screening for peptide modulator compounds (coding sequence - XXX) interacting with a known membrane protein. Peptides influencing this membrane protein can have their modulatory effects measured.
Figure 6 - *Schematic diagram showing phenotype investigation of peptide randomisation of a larger protein - cellular modulators.* A low efficiency modulator has been randomised to increase its binding efficiency of a known membrane protein. X is a randomised construct from the DNA library.
Figure 7 *- Schematic diagram showing phenotype investigation of peptide randomisation of a larger protein - receptors - e.g. ScFv.* ScFV binding site randomisation (altered regions indicated by X) can influence the binding affinity, such affinity can then be phenotypically measured.

### Examples

### Example 1- Description of the preferred embodiment

### Prophetic example: Construction of a genomic library expressed in a cell line

The aim of this experiment could be to improve the binding of a given ligand to a specific ion channel. This is done by randomising specific amino acids within the ion channel in which the ligand is known to bind, introducing these randomised gene products into cells and test it in the micro-array system. Examples of micro-array tests are shown in example 3,4,5.
The ion channel gene is inserted into a bicistronic retroviral vector containing all the retroviral *cis*-elements necessary for transducing target cells as well as an internal ribosome entry site from EMCV followed by a neomycin selection gene (Morgan RA, Couture L, Elroy-Stein O, Ragheb J, Moss B, Anderson WF.Retroviral vectors containing putative internal ribosome entry sites: development of a polycistronic gene transfer system and applications to human gene therapy. NAR 1992, 20:1293-9). When the ion channel gene is inserted upstream of the IRES element both the ion channel gene and the neo-gene will be found on the same mRNA transcripts arising from this retroviral vector transcription unit. The ion channel gene will then be translated by a cap-scanning mechanism while the neo translation will be initiated from the IRES element. Randomisation of four amino acids in the ion channel is performed by a two-step PCR-based method (see figure 1) (Efficient non-PCR-mediated overlap extension of PCR fragments by exonuclease "end polishing". Jespersen T, Duch M, Pedersen FS Biotechniques 1997 Jul;23(1):48, 50, 52). In the first step two parallel PCR's, amplifying each end of the transcription unit (retroviral vector), is performed. Primer #2 encodes 12 randomised nucleotides (given rise to 4 amino acids) just after the 3' sequence annealing to the DNA strand. When these randomised nucleotides are incorporated in the coding sequence of the ion channel up to 204 different genotypes will be constructed. Primer #2 and #3 are complementary to each other in the 5'end of the primer and sequences introduced by these primers can thereby by used in an overlap extension procedure. PCR should be performed with the following components: 0.5 µM primers (oligonucleotides), 100 ng template/100 µl reaction, 2.5 unit proofreading polymerase enzyme (such as Pfu (Stratagene) or Platinum Taq DNA polymerase High Fidelity (Life Technologies)) with the corresponding buffer and 200 µM dNTP. PCR conditions (15 cycles): 95 °C 30 s, 50 °C 15 s, 72 °C 3 min, preceded by 2 minutes at 95 °C and followed by 5 minutes at 72 °C.
The genomic library obtained in the overlap extension procedure is introduced into the target cells through retroviral packaging cells, such as BOSC23 (Pear WS, Nolan GP, Scott ML, Baltimore D. Production of high-titer helper-free retroviruses by transient transfection. Proc Natl Acad Sci U S A. 1993 Sep 15;90(18):8392-6)(Coffin JM (1996) Retroviridae). When the PCR products is transfected (with e.g. lipofectamine (Life Technologies), according to manufactures instructions) into the packaging cell line the DNA will be transcribed into RNA which will be packed into the retroviral particles produced by the packaging cells. 72 hours post transfection medie should be filtered (0.5 µm filter) and added directly to the target cells (transduction). 48 hours post-transduction selection (neomycin) media should be added to the cells. After 14 days non-transduced cells will be dead and transduced cells will appear as colonies. The colonies should be mixed (trypsinised) to obtain a population containing the genomic library. This population can now be used either immediately in screening experiments or be stored at -80 degrees.

### Example 2 - Chip for Phenotype analysis

A suitable chip for patch clamp experiments is described in WO 02/29402. For use in this invention the chip is modified to enable the extraction of mRNA from the cell and is as shown in figure 3.

The chip contains a planar substrate comprising a first surface part and an opposite second surface part, the first surface part having a plurality of sites each of which is adapted to hold an ion channel-containing structure.

Each site comprises a measuring electrode associated therewith, the substrate carrying one or more reference electrodes, the measuring electrodes and the respective reference electrode or reference electrodes being electrodes capable of generating, when in electrolytic contact with each other and when a potential difference is applied between them, a current between them by delivery of ions by one electrode and receipt of ions by the other electrode.

Each of the sites is adapted to provide a high electrical resistance seal between an ion channel-containing structure held at the site and a surface part of the site. The seal, when provided, separates a domain defined on one side of the ion channel-containing structure and in electrolytic contact with the measuring electrode from a domain defined on the other side of the ion channel-containing structure and in electrolytic contact with the respective reference electrode. The seal allows a current flowing through ion channels of the ion channel-containing structure between the electrodes to be determined and/or monitored. The electrodes of the invention are integrated with the substrate and having been formed by a wafer processing technology.

An ion channel-containing structure (e.g. an animal cell) in a solution may be guided towards a site on a substrate either by active or passive means. When the ion channel-containing structure makes contact with the site, e.g. substrate around an electrode, the contact surfaces form a high electrical resistance seal (a giga-seal) at the site, e.g. surrounding the electrode, so that an electrophysiological property of the ion channels can be measured using the respective electrode. Such electrophysiological property may be current conducted through the part of membrane of the ion channel-containing structure that is encircled by the giga-seal.

In the present context, the term "giga-seal" normally indicates a seal of a least 1G ohm, and this is the size of seal normally aimed at as a minimum, but for certain types of measurements where the currents are large, lower values may be sufficient as threshold values.

Preferably the whole cell configuration method of patch-clamping is used. This configuration may be obtained by applying, between the measuring electrode associated with each approved site and a reference electrode, a series of second electric potential difference pulses, monitoring a second current flowing between the measuring electrode and the reference electrode, and interrupting the series of second electric potential difference pulses whenever said second current exceeds a predetermined threshold value, thereby rupturing the part of the ion channel-containing structure which is closest to the measuring electrode.

Alternatively, the whole-cell configuration may be obtained by subjecting the part of the ion channel-containing structure that is closest to the measuring electrode to interaction with a pore forming substance.

It should be noted that in the present context, the term "whole-cell configuration" denotes not only configurations in which a whole cell has been brought in contact with the substrate at a measuring site and has been punctured or, by means of a pore-forming substance, has been opened to electrical contact with the cell interior, but also configurations in which an excised cell membrane patch has been arranged so that the outer face of the membrane faces "upwardly", towards a test sample to be applied.

As the measuring electrode associated with a site is one of a plurality of electrodes on the substrate, and the ion channel-containing structure is one of many in a solution, it is preferable that a plurality of measuring sites exists on a particular substrate. The plurality of measuring sites enables a plurality of test cells to be analysed simultaneously.

### Example 3 - Electrophysiological measurements

Electrophysiological measurements can be performed using a chip construct made of the substrate and used as described above and in figure 3, to perform patch clamp testing by applying voltage changes to the test cell.

A typical measurement comprises adding a specific test sample to a measuring set-up, for which reason each measuring set-up is separated from other measuring set-ups to avoid mixing of test samples and electrical conduction in between said set-ups. Additionally control cells that have not been transformed with a heterologous DNA sequence are tested to provide a comparative phenotype measurement for that cell line.

A number of test cells can be studied at any one time and the electrophysiological measurements made and analysed to indicate which if any, of the test cells exhibit phenotypes different to that of the control cells.

Electrophysiological measurements produced through the application of voltage changes to the test cell, are used to analyse one or more from the following cellular phenotype characteristics: current amplitude (the maximal current measured during a depolarisation pulse), activation kinetics (the time constants which the current increases during a depolarisation pulse), inactivation kinetics (the time constants which the current decreases during a depolarisation pulse), deactivation kinetics (the time constants which the current decreases after a depolarisation pulse), activation threshold (the voltage potential opening the ion-permeable pores in the cell membrane) and tail current (the current measured after a depolarisation pulse)(Hille B. Ion channels of excitable membranes. 3th edition. 2001. Sinauer Associates, Inc, Sunderland, MA, USA).

### Example 4 - Fluorescence based screening

Certain fluorescent probes are known to bind cellular molecules to indicate one or more of a number of cellular parameters including but not limited to pH (e.g. H+ specific probes), membrane potential and concentration fluxes of ions (e.g. Ca²⁺ (fura2) and Mg²⁺ (mag-fluo4) specific probes (Molecular Probes, Inc, The Netherlands))(Methods Cell Biol 1989;30:157-92 Bright GR, Fisher GW, Rogowska J, Taylor DL. Fluorescence ratio imaging microscopy)(Inoue S. Progress in video microscopy. Cell Motil Cytoskeleton 1988;10(1-2):13-7

When voltage changes are applied to the test cell situated on the substrate, where fluorescent probes have been additionally added, the fluorescent probe binds to its specific target molecule, if present within the test cell, causing the induction of a fluorescent signal. The fluorescent signal can be quantified by fluorescence microscopy (Methods Cell Biol 1989;30:157-92 Bright GR, Fisher GW, Rogowska J, Taylor DL. Fluorescence ratio imaging microscopy)(Inoue S. Progress in video microscopy. Cell Motil Cytoskeleton 1988;10(1-2):13-7) and thereby translated into a direct measurement of the intracellular condition being measured such that and the test cell is compared to control cells to identify those test cells exhibiting different phenotypes.

### Example 5 - Size or shape measurements

Measurement of changes in size or shape of cells expressing the DNA library enables the characterisation of polynucleotides and/or genes and/or peptides and/or polypeptides and/or proteins that have influenced the overall physical conformation of the host cell in comparison to the control cells. The measurements are performed by automatic microscopic detection (Foskett JK. [Ca2+]i modulation of Cl- content controls cell volume in single salivary acinar cells during fluid secretion. Am J Physiol 1990 Dec;259(6 Pt 1):C998-100:1)(S Muallem, BX Zhang, PA Loessberg, and RA Star Simultaneous recording of cell volume changes and intracellular pH or Ca2+ concentration in single osteosarcoma cells LTMR-106-01. J. Biol. Chem. 1992 267: 17658-17664)

### Example 6 - Isolation of mRNA and characterisation of a "hit"

Cells providing a "positive" result or "hit", are those cells that exhibit an altered phenotype compared to that of untransformed control cells from the same cell line.

When a positive cell has been identified, characterisation of the genotype is the next step in the screening of heterologous DNA sequences. Characterisation of the genotype underlying a positive phenotypic signal will be analysed by isolating the mRNA from the positive cell. The mRNA can be extracted by one or more methods from pipetting, cell lysis and mechanical removal.

Pipetting occurs whereby one of a pipette, syringe, needle or similar tool is inserted into the cell and part of the cytosol is extracted. A preferable method of pipetting is achieved by extraction of cell cytosol through the channel at the cell capture site {Figures 2 & 3} of the test substrate of the chip. Further preferably the removal of mRNA through said channel is achieved by suction exerted by the micropumps (9) or the insertion of one of a pipette, syringe, needle or similar tool into the cell after being passed through said channel.

Lysis is achieved by the addition of a lysis buffer (e.g. 0.2 M NaOH, 1 % SDS) to the chamber containing the cell, thereby breaking the cell membrane apart and releasing the cellular components.

The mechanical method of mRNA extraction is conducted by; scraping the cell out of the chamber using a tool specially designed for removal of the cell from the hole in the array. The process of scraping the cell from the chamber ruptures the cell and releases the cellular contents from which the mRNA is thereafter extracted.

Further characterisation of the polynucleotide, peptide, polypeptide and/or protein influenced by the applied stimulus is performed by RT-PCR (Reverse Transcriptase PCR) characterisation of the mRNA isolated from the test cell.

In order to amplify only the nucleotides of interest, RT-PCR primer design requires prior knowledge of the nucleotide sequences of interest or those flanking the nucleotides of interest. In this invention the regions flanking the nucleotide of interest are known, as they comprise the regions of the viral vector surrounding the heterologous DNA sequence.

After mRNA has been isolated from the positive cell, the RT-PCR primers designed to be complementary to the viral regions flanking the nucleotides of interest, are used to undertake a reverse transcription reaction to synthesise a single stranded cDNA. This cDNA is further used as the template in a conventional PCR reaction, the primers for which contain known restriction sites so that the double stranded products from PCR can be easily subcloned into a further vector.

This new vector containing the nucleotides of interest can be used in sequencing reactions and in expression of appropriate products, in order to better characterise the nucleotide of interest and if appropriate its expression products. Further characterisation of the expression products can be achieved by, for example, conventional patch-clamp or fluorescence testing.

Analysis of cellular expressed cDNA libraries on the chip technology enables the screening of a plurality of known and unknown proteins. The parameters employed in a specific screen are adjusted to the protein function that is under investigation, e.g. if the screening is for new voltage-gated ion channels, the screening method will preferably involve electrophysiological parameters.

### Example 7-Application of a test agent

The preferred embodiment can further include a step whereby the test cells are exposed to a test agent, which may or may not influence the phenotype in conjunction with the heterologous DNA sequence. The phenotypes of test cells are compared to control cells that have additionally been exposed to the test agent.

Preferably the test agent is at least one from: small organic molecules, small peptides, neurotransmitters, hormones and cytokines.

### Example 8 - Further applications of the invention

The primary use of the invention is in screening DNA libraries, the applications of which are numerous. Potential applications of the invention include, but are not limited to identification and/or characterisation and/or synthesis of novel therapeutic compounds and novel drug targets.

Further applications include the identification and/or characterisation of: molecules involved in or influencing cellular cascade and/or transduction pathways; amino acids of a binding protein involved and/or necessary for binding to a target molecule

A yet further application of the invention is the screening of randomised and/or polymorphic libraries for identification and/or characterisation of variants of improved and/or reduced function.

### Example 9 - Randomised libraries as a starting point

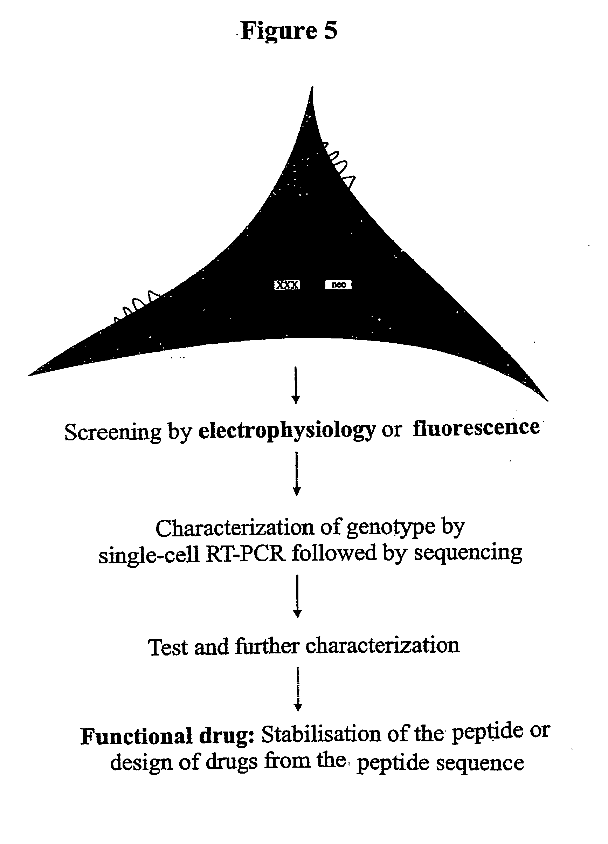

Expression of randomised peptides is of major interest both for the potential discovery of new drugs and in the study of the function and interaction patterns of small peptides {Figure 5}.

Protein function and cellular pathways can be modified by certain small peptides by their binding to specific peptide domains within proteins. As such small peptides possess the potential ability to treat disease. Further study of such peptides found in this invention may show an in vivo lack of function. However, from the isolated mRNA and expressed products it is further possible to create a structural model of the peptide from which a synthetic organic compound mimicking the function of the peptide can be created.

Furthermore, a number of small peptides of unknown function have been found both extra- and intra-cellularly in vivo. Expression of randomised peptides will be one method to elucidate the function of such small peptides.

### Example 10 - Larger protein randomisation libraries as a starting point

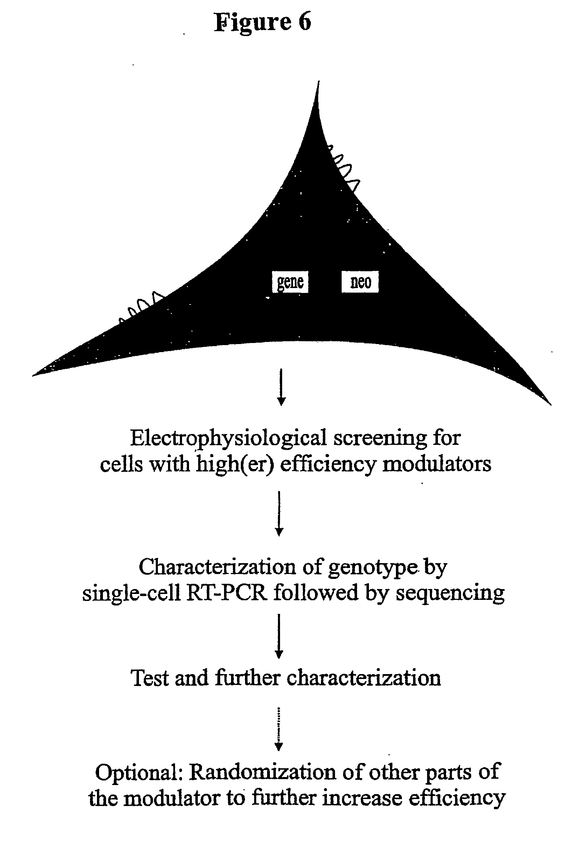

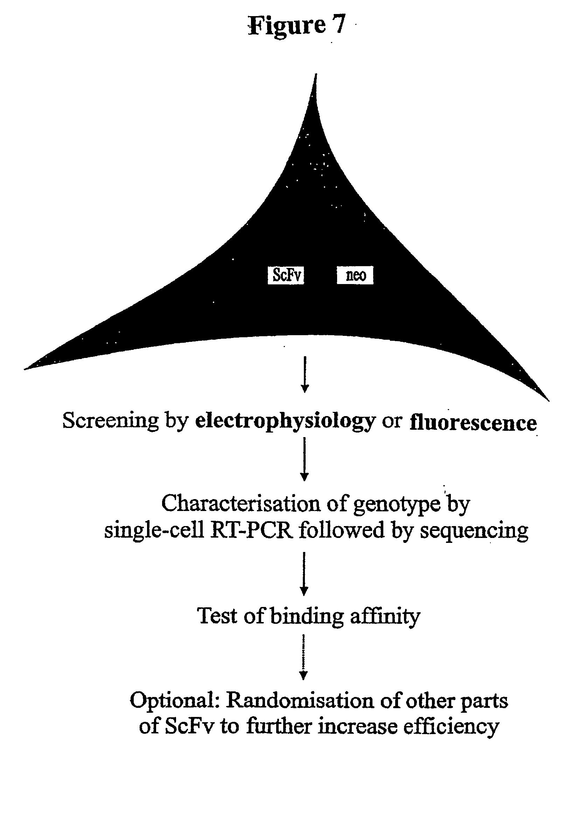

Further randomisation libraries can comprise the randomisation of localised peptide domains within a larger protein {Figures 6 & 7}. Binding proteins contain domain with different functions, i.e. a binding domain and usually an effector domain. A number of such binding proteins are known to bind their targets with a relatively low affinity, and therefore randomisation of the binding domain to increase (e.g. antibodies) or decrease (e.g. reduce side effects) the affinity between the ligand and target proteins produces a desirable library to study.

### Example 11 - Polymorphic libraries as a starting point

Many genes present in the human population are polymorphic. Polymorphic libraries are of interest in screening of potential drugs for side effects and differentiated response pattern. When the polymorphic library is large and/or not all polymorphic sequences are known it may be beneficial to perform screenings by the chip technology for the ease of convenience of studying.

## Claims

1. A method for performing electrophysiological measurements comprising the steps of:
(i) providing a substrate for making the electrophysiological measurements upon which at least one cell can be arranged;
(ii) providing a plurality of cells, each cell comprising a different heterologous DNA sequence derived from a DNA library, wherein each cell expresses the heterologous DNA sequence it comprises;
(iii) arranging the plurality of cells provided in step (ii) on the substrate to permit detection and/or measurement of a change (in comparison to a control cell) in the electrophysiology of each cell, said change being a result of expression of the heterologous DNA sequence, and
(iv) identifying at least one cell of interest which shows at least one phenotypic change,
**characterised in that**, the method comprises the further steps of:
isolating the cell of interest, and/or genetic material therefrom; and
isolating mRNA from the cell of interest identified in step (iii).

2. A method as claimed in Claim 1 wherein the method further comprises the step of sequencing the genetic material.

3. A method as claimed in Claim 2 wherein the method further comprises the step of storing or recording the sequence information on an information carrier, such as a computer disk.

4. A method as claimed in any previous Claim wherein the DNA library is a cDNA library.

5. A method as claimed in any one of the preceding claims wherein the change in the electrophysiology of the cell is detected and/or measured by patch clamping.

6. A method as claimed in any preceding claim wherein the cell is treated with a test agent before step (iii).

7. A method as claimed in Claim 6 wherein the test agent is selected from at least one of the following: small organic molecules, small peptides, neurotransmitters, hormones and cytokines.

8. A method as claimed in any preceding claim wherein the cell is an animal cell.

9. A method as claimed in any preceding claim wherein the animal cell is selected from: Human Embryonic Kidney 293 (HEK293), Chinese Hamster Ovary (CHO), COS, MDCK, NG108, NIH3T3 or T84.

10. A method as claimed in any previous claim wherein the cells are arranged at spaced-apart locations in or on the substrate.

## Revendications

1. Procédé de réalisation de mesures électrophysiologiques comprenant les étapes consistant à :
(i) fournir un substrat pour réaliser les mesures électrophysiologiques sur lequel au moins une cellule peut être disposée ;
(ii) fournir une pluralité de cellules, chaque cellule comprenant une séquence d'ADN hétérologue différente, issue d'une banque d'ADN, chaque cellule exprimant la séquence d'ADN hétérologue qu'elle comprend ;
(iii) disposer la pluralité de cellules fournies dans l'étape (ii) sur le substrat pour permettre la détection et/ou la mesure d'un changement (par rapport à une cellule témoin) dans l'électrophysiologie de chaque cellule, ledit changement étant le résultat de l'expression de la séquence d'ADN hétérologue ; et
(iv) identifier au moins une cellule intéressante présentant au moins un changement phénotypique,
**caractérisé en ce que** le procédé comprend les étapes supplémentaires consistant à :
isoler la cellule d'intérêt et/ou son matériel génétique ; et
isoler l'ARNm de la cellule d'intérêt identifiée dans l'étape (iii).

2. Procédé selon la revendication 1, dans lequel le procédé comprend en outre l'étape de séquençage du matériel génétique.

3. Procédé selon la revendication 2, dans lequel le procédé comprend en outre l'étape de stockage ou d'enregistrement des informations sur les séquences sur un support d'informations, tel qu'un disque d'ordinateur.

4. Procédé selon l'une quelconque des revendications précédentes, dans lequel la banque d'ADN est une banque d'ADNc.

5. Procédé selon l'une quelconque des revendications précédentes, dans lequel le changement dans l'électrophysiologie de la cellule est détecté et/ou mesuré par "patch clamp".

6. Procédé selon l'une quelconque des revendications précédentes, dans lequel la cellule est traitée avec un agent d'essai avant l'étape (iii).

7. Procédé selon la revendication 6, dans lequel l'agent d'essai est choisi parmi au moins l'un des éléments suivants : les petites molécules organiques, les petits peptides, les neurotransmetteurs, les hormones et les cytokines.

8. Procédé selon l'une quelconque des revendications précédentes, dans lequel la cellule est une cellule animale.

9. Procédé selon l'une quelconque des revendications précédentes, dans lequel la cellule animale est choisie parmi : les cellules embryonnaires rénales humaines 293 (HEK293), d'ovaire de hamster chinois (CHO), COS, MDCK, NG108, NIH3T3 ou T84.

10. Procédé selon l'une quelconque des revendications précédentes, dans lequel les cellules sont disposées à des emplacements espacés dans ou sur le substrat.

## Patentansprüche

1. Verfahren zur Durchführung von elektrophysiologischen Messungen, das folgende Schritte umfasst:
(i) Bereitstellen eines Substrats für die Durchführung der elektrophysiologischen Messungen, auf dem zumindest eine Zelle angeordnet werden kann,
(ii) Bereitstellen einer Vielzahl von Zellen, von denen jede eine andere heterologe DNA-Sequenz aufweist, die von einer DNA-Bibliothek abgeleitet ist, wobei jede Zelle die heterologe DNA-Sequenz exprimiert, die sie enthält,
(iii) Anordnen der Vielzahl von im Schritt (ii) bereitgestellten Zellen auf dem Substrat, um eine Detektion und/oder Messung einer Änderung im Vergleich zu einer Vergleichszelie in der Elektrophysiologie einer jeden Zelle zu ermöglichen, wobei diese Änderung ein Ergebnis der Expression der heterologen DNA-Sequenz ist, und
(iv) Identifizieren von wenigstens einer interessierenden Zelle, die wenigstens eine phänotypische Änderung zeigt,
**dadurch gekennzeichnet, dass** das Verfahren weiterhin die Schritte umfasst,
isolieren der interessierenden Zelle und/oder von genetischem Material, das von ihr stammt, und Isolieren von mRNA von der im Schritt (iii) identifizierten interessierenden Zelle.

2. Verfahren nach Anspruch 1, das weiterhin den Schritt der Sequenzlerung des genetischen Materials umfasst.

3. Verfahren nach Anspruch 2, das weiterhin den Schritt des Speicherns oder Aufzeichnens der Sequenzinformation auf einem Informationsträger, wie z.B. einer Rechnerdiskette umfasst.

4. Verfahren nach einem der vorhergehenden Ansprüche, bei dem die DNA-Bibliothek eine cDNA-Bibliothek ist.

5. Verfahren nach einem der vorhergehenden Ansprüche, bei dem die Änderung in der Elektrophysiologie der Zelle durch Patch Clamping erkannt und/oder gemessen wird.

6. Verfahren nach einem der vorhergehenden Ansprüche, bei dem die Zelle vor dem Schritt (iii) mit einem Test-Wirkstoff behandelt wird.

7. Verfahren nach Anspruch 6, bei dem der Test-Wirkstoff wenigstens einer aus der folgenden Gruppe ist: Kleine organische Moleküle, kleine Peptide, Neurotransmitter, Hormone und Zytokine.

8. Verfahren nach einem der vorhergehenden Ansprüche, bei dem die Zelle eine tierische oder menschliche Zelle ist.

9. Verfahren nach einem der vorhergehenden Ansprüche, bei dem die tierische oder menschliche Zelle eine der folgenden Zellen ist: Menschliche embryonale Nierenzelle 293 (HEK293), Elerstockzelle des chinesischen Hamsters (CHO), COS, MDCK, NG108, NIH3T3 oder T84.

10. Verfahren nach einem der vorhergehenden Ansprüche, bei dem die Zellen an voneinander beabstandeten Orten in oder auf dem Substrat angeordnet sind.
